# EUROPEAN PATENT APPLICATION

(11) **EP 4 566 637 A1**
(43) Date of publication of application: **11.06.2025**
(21) Application number: 24020049.3
(22) Date of filing: 02.02.2024
(51) Int. Cl.: A61L 27/18, A61L 27/28, A61L 27/46

(54) **BIODEGRADABLE 3D PRINTED STRUCTURE FOR BONE AUGMENTATION**

(30) Priority: 07.12.2023 HR P20231599; 13.12.2023 WO PCT/HR2023/050005
(71) Applicant: TOPOMATIKA d.o.o., 10431 Sveta Nedelja (HR)
(72) Inventor: IVANKOVIC, Hrvoje, 10291 Prigorje Brdovecko (HR); IVANKOVIC, Marica, 10291 Prigorje Brdovecko (HR); ROGINA, Anamarija, 10000 Zagreb (HR); BAUER, Leonard, 10340 Vrbovec (HR); RESSLER, Antonia, 48000 Koprivnica (HR); MILOVAC LERGA, Dajana, 23000 Zadar (HR); SECERKADIC, Amir, 10000 Zagreb (HR); HERCIGONJA, Tomislav, 10000 Zagreb (HR)
(74) Representative: Strniscak, Tomislav

(57) **Abstract**

Biodegradable 3D printed structure for bone augmentation (1) comprises an outer structure (100) and an inner structure (200) that are printed from polylactide (PLA), wherein the inner structure (200) is filled with a chitosan and hydroxyapatite gel and the outer structure (100) is coated with a chitosan and hydroxyapatite composite. The outer structure (100) has a tunnel-like shape resembling an elongated projection of the letter "U" rotated 180 degrees in depth. Implants made in the form of a biodegradable 3D-printed structure for bone augmentation as disclosed in the present invention can be adapted to each individual patient and each individual situation, which will optimize the dentist's time required for the procedure and treatment, and will also facilitate the recovery of the patient due to the reduction of the invasiveness of the procedure. This makes the entire procedure faster, simpler, and painless, but also financially acceptable for patients.

## Description

### FIELD OF THE INVENTION

The present invention relates to biodegradable structures for bone augmentation, more specifically those obtained through 3D printing.

### TECHNICAL PROBLEM

Thousands of surgical procedures are performed daily to replace or regenerate tissue damaged by disease or injury. The main challenge for bone tissue engineering is the development of 3D porous structures as biological substitutes that restore, maintain, or improve the function of damaged tissue.

In practice, titanium implants are most often used, which are removed due to their inert nature after a certain time required for bone augmentation (approximately 6 months), which causes certain trauma to the patient as well as additional stress to the newly formed tissue.

The inventor aimed to find a new structure for bone augmentation that would be cheaper, that would involve fewer complications and that would result in a faster and less stressful treatment procedure for the patient.

### PRIOR ART

The biggest problem in dental implantology is the lack of the necessary volume of the jaw bone, which atrophies after tooth extraction. Bone loss after tooth extraction, inflammation, or trauma requires an oral surgical procedure for bone augmentation. An adequate bone volume at the desired location is required for the placement of an endosseous implant. During bone ridge augmentation, it is desirable that bone regeneration occurs as quickly as possible. This is achieved through guided bone regeneration (GBR) with autologous or artificially derived bone from synthetic or animal-derived materials. The basic principle of the GBR method is to prevent tissue ingrowth into a bone defect by applying a 3D porous structure to restore the periodontium and to stimulate vascularization for the growth of new bone tissue. The success of the GBR method depends on the properties of porous structures that have to be biocompatible and mechanically resistant to provide sufficient space for tissue regeneration and should be resistant to everyday mechanical burdens inside the oral cavity. Additionally, porous structures should integrate with the surrounding tissue and be easily shaped to match the size and shape of the treated area. Previous periodontal treatments employ various inert structures such as titanium which is widely available in clinical applications due to the reduction of the patient's immune response and mechanical resistance.

However, the main disadvantage of titanium implants is the secondary surgical procedure to remove them, with a risk of infection. According to the inventor's knowledge, a small number of studies have been dedicated to additive technologies such as 3D printing for the production of porous synthetic biodegradable structures. By modulating the structure of biodegradable implants, synthetic materials provide strengths in the order of magnitude of natural bone tissue. In the prior art, the inventor determined the existence of the US patent application US20220403327A1 for the invention entitled " *Customizable 3D cell culture system comprising hydrogel-embedded cells and uses thereof".* The first patent claim indicates that it is a 3D structure comprising a first layer consisting of a solid porous polymer structure containing the first cell type and a second layer consisting of a biocompatible hydrogel with the second cell type, which is in contact with the first layer, which descriptively corresponds to the fact that the first layer is coated with the second layer.

Furthermore, the type of biocompatible polymer from which the first layer is made, including PLA, is disclosed in patent claim 8. The use of a biocompatible hydrogel that may contain chitosan and hydroxyapatite is also mentioned in the description of the invention.

Nowhere does that description of the invention specify the shape of the 3D printed structure that would at least be similar to or correspond to the shape and the printed structure disclosed herein.

Through the prior art search, the inventor hasn't encountered, nor is it known to him that there might be a solution that would disclose the biodegradable 3D printed structure for bone augmentation with the shape and dimensions as outlined in the present invention.

### BRIEF SUMMARY OF THE PRESENT INVENTION

Numerous studies indicate that the biocompatibility and bioactivity of synthetic biodegradable polymers is lower compared to natural polymers and bioactive materials used in bone tissue regeneration. Bioactivity as well as faster tissue regeneration in clinical practice is most often achieved by adding various growth factors such as BMP-2, but is controversial due to the high concentrations added to ensure their effect. As an alternative to growth factors, the inventor investigated the application of bioactive calcium phosphate ceramics (hydroxyapatite) that is chemically and structurally similar to the mineral phase of natural bone. The present invention is based on the development of biodegradable and bioactive implants and their fabrication and shaping by 3D printing, as an alternative to inert titanium structures for bone ridge augmentation. The new 3D biodegradable structure is based on biodegradable polymer and hydroxyapatite as well as fabrication technology for the creation of an individualized implant for bone tissue augmentation. The developed structure represents a replacement for the previous metal implants that are most commonly used in the form of titanium mesh. Namely, by applying a newly developed implant, with full functionality, a secondary surgical procedure is avoided, since over time the implant will be resorbed in the body. Implants of a predetermined shape and dimensions that suit each individual patient and each individual situation will optimize the dentist's time and also facilitate the recovery of the patient due to the reduction of the invasiveness of the procedure. This makes the entire procedure faster, simpler, and painless, but also financially acceptable for patients. The material is threecomponent and consists of polylactide (PLA)-medical, thermoplastic biodegradable polymer with suitable mechanical properties, whose printing produces a 3D porous structure of defined pores and shapes suitable for vascularization and formation of a new tissue, it is filled or coated with a hydrogel or a mixture of chitosan and hydroxyapatite. The first tests showed that this 3D structure of PLA has good mechanical properties, while the content i.e. the fact that the structure is coated with a composite of chitosan and hydroxyapatite accelerates bone regeneration. Inert titanium meshes that adapt to the shape and size of the defect with the help of additive manufacturing technologies have been used in previous periodontal treatments. The treatment involves two steps, filling the bone defect with a suitable augmentation material (defect filling), and then closing the defect with a titanium mesh that is covered with a thin membrane. Upon completion of bone augmentation, the titanium mesh is removed with an additional surgical procedure followed by the placement of a dental implant. The 3D printed structure disclosed in the present invention replaces the titanium mesh and its function of protecting the bone defect during bone repair with simultaneous degradation. The shape, size, and microstructure of the outer part are adapted to the defect requirements according to the desired degradation rate and mechanical properties. The inner (lower) part replaces the defect-filling material, accelerating bone formation. The inner part is shaped using 3D printing to match the size and shape of the defect and is made of biodegradable thermoplastic material as a highly porous 3D structure. This structure is then filled with a biodegradable hydrogel containing a bioactive inorganic component to accelerate bone growth. The biodegradable 3D printed bone augmentation structure disclosed in the present invention comprises an outer structure and an inner structure wherein both structures are printed from polylactide (PLA). The inner structure is filled with a chitosan and hydroxyapatite gel, while the outer structure is coated with a chitosan and hydroxyapatite composite. The outer structure has a tunnel-like shape resembling an elongated projection of the letter "U" rotated 180 degrees in depth. The front and the rear side of the outer structure are connected by "X"-shaped connections. Typically, there are five such connections, and when viewed from the lateral side of the outer structure, the interior formed by the tunnel shape of the outer structure is visible. The inner structure contains multiple perforated plates printed one above the other so that they are bordered by the outer structure.

The inner structure contains both an internal raster and struts, whereby the struts support and hold the gap between the two perforated plates so that the internal raster is formed. The inner structure extends longitudinally along the axis of the tunnel, with the axis orientation from the front to the rear of the outer structure, has a width of at least 2/3 of the width of the tunnel and extends through the entire height and length of the outer structure and is firmly connected to the inner vault of the tunnel formed by the outer structure.

### BRIEF DESCRIPTION OF THE DRAWINGS

The accompanying drawings which are incorporated in the description, and which form a part of the invention description, illustrate so far, the best-considered embodiment of the invention and help to explain the basic principles of the invention. This invention is not in any way limited by drawings that are an integral part of the present invention.

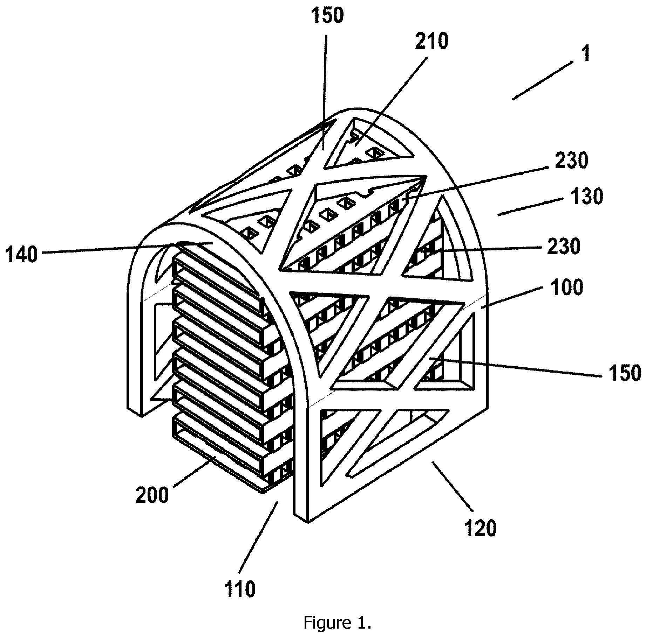

Figure 1. A view of the biodegradable 3D printed structure for bone augmentation without filling

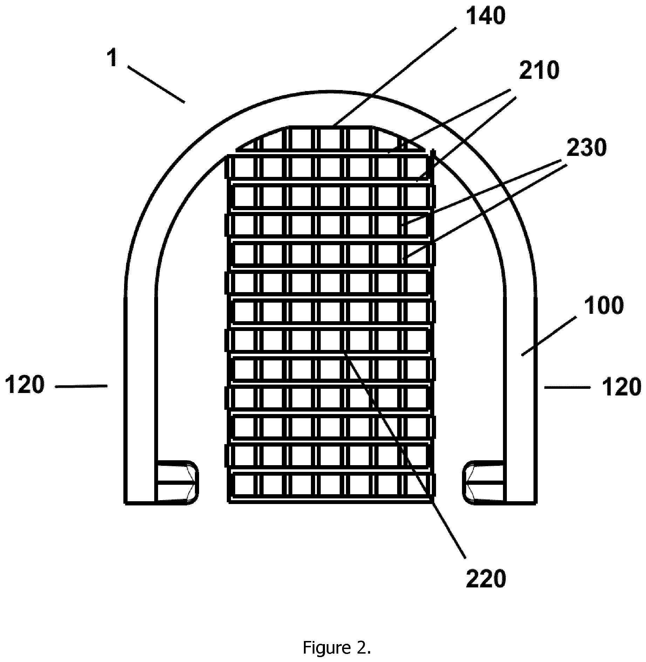

Figure 2. Front view of the biodegradable 3D printed structure for bone augmentation

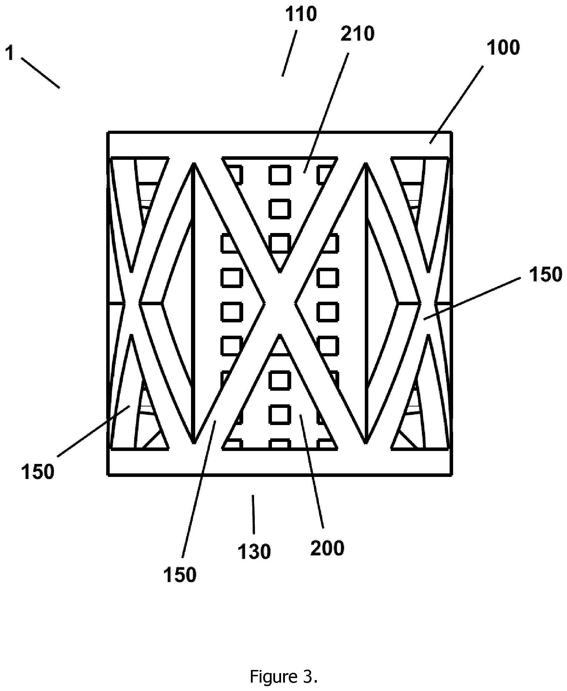

Figure 3. Lateral view of the biodegradable 3D printed structure for bone augmentation

### A LIST OF REFERENCE SIGNS USED IN DRAWINGS

1 - biodegradable 3D printed structure for bone augmentation
100 - outer structure
110 - front side of the outer structure
120 - lateral side of the outer structure
130 - rear side of the outer structure
140 - inner tunnel vault
150 - "X"-shaped connection
200 - inner structure
210 - perforated plate
220 - internal raster
230 - strut

### DETAILED DESCRIPTION OF AT LEAST ONE WAY OF CARRYING OUT THE INVENTION

Biodegradable 3D printed structure for bone augmentation 1 disclosed herein is printed with a 3D printer using polylactide (PLA). Essentially, both parts of this structure, the outer structure 100 and the inner structure 200 are printed at the same time. After structure 1 is printed, the inner structure 200 is filled with a chitosan and hydroxyapatite gel, while the outer structure 100 is coated with a chitosan and hydroxyapatite composite. The outer structure 100 is printed in the shape of a tunnel in the form of an elongated projection of the letter "U" rotated 180 degrees in depth, where the outer structure 100 contains the front side of the outer structure 110 and the rear side of the outer structure 130, as well as connections in the form of the letter "X" 150 and two lateral sides of the outer structure 120, all achieved by printing on a 3D printer. The front side of the outer structure 110 and the rear side of the outer structure 130 are connected by at least five "X"-shaped connections 150 so that when viewed from the lateral side of the outer structure 120, the interior formed by the tunnel shape of the outer structure 100 is visible. In one common embodiment dimensions of the outer structure are as follows: the width of the outer structure 100 in the part that forms the base of the letter "U" rotated 180 degrees is 20 mm, the height of the outer structure 100 is 20 mm, wherein the distance between the front side of the outer structure 110 and the rear side of the outer structure 130 is 20 mm. The inner structure 200 itself contains multiple perforated plates 210 printed one above the other so that they are bordered by the outer structure 100. The inner structure 200 also contains an internal raster 220 and struts 230, wherein the struts 230 support and hold the gap between the two perforated plates 210, so that the internal raster 220 is formed. The inner structure extends longitudinally along the axis of the tunnel, with the axis orientation from the front of the outer structure 110 towards the rear of the outer structure 130, has a width of at least 2/3 of the width of the tunnel and extends through the entire height and length of the outer structure 100 and is firmly connected to the inner vault of the tunnel 140 which is formed by the outer structure 100.

## Claims

1. Biodegradable 3D printed structure for bone augmentation (1) **characterized in that** it comprises an inner structure (100) and an outer structure (200) wherein both structures are printed from polylactide (PLA) and wherein the inner structure (200) is filled with a chitosan and hydroxyapatite gel, while the outer structure (100) is coated with a chitosan and hydroxyapatite composite.

2. Biodegradable 3D printed structure for bone augmentation (1) **characterized in that** the outer structure (100) has a tunnel-like shape resembling an elongated projection of the letter "U" rotated 180 degrees in depth, wherein the outer structure (100) comprises the front side of the outer structure (110) and the rear side of the outer structure (130), "X"-shaped connections (150) as well as two lateral sides of the outer structure (120), wherein the front side of the outer structure (110) and the rear side of the outer structure (130) are connected by at least five connections in the shape of the letter "X" (150), so that when viewed from the lateral side of the outer structure (120), the interior formed by the tunnel shape of the outer structure (100) is visible.

3. Biodegradable 3D printed structure for bone augmentation (1) as claimed in claim 2 **characterized in that** the dimensions of the outer structure are as follows: the width of the outer structure (100) in the part that forms the base of the letter "U" rotated by 180 degrees is 20 mm, the height of the outer structure (100) is 20 mm as well, wherein the distance between the front side of the outer structure (110) and the rear side of the outer structure (130) is 20 mm.

4. Biodegradable 3D printed structure for bone augmentation (1) as claimed in any of the previous patent claims **characterized in that** the inner structure (200) comprises multiple perforated plates (210) printed one above the other so that they are bordered by the outer structure (100), furthermore the inner structure (200) comprises both an internal raster (220) and struts (230), wherein the struts (230) support and hold the gap between the two perforated plates (210), so that the internal raster (220) is formed, whereby the inner structure extends longitudinally along the axis of the tunnel, with the axis orientation from the front side of the outer structure (110) to the rear side of the outer structure (130), has a width of at least 2/3 of the width of the tunnel and extends through the entire height and length of the outer structure (100) and is firmly connected to the inner vault of the tunnel (140) formed by the outer structure.
